# EUROPEAN PATENT APPLICATION

(11) **EP 1 647 293 A1**
(43) Date of publication of application: **19.04.2006**
(21) Application number: 04255693.6
(22) Date of filing: 18.09.2004
(51) Int. Cl.: A61M 5/32

(54) **Safety syringe having retraction mechanism**

(71) Applicant: Intai Technology Corp., Taichung City 407 (TW)
(72) Inventor: Lou, Jin-Rong, Taichung City 407 (TW)
(74) Representative: Brown, Michael Stanley

(57) **Abstract**

A safety syringe includes a barrel, a connector securely seated in the barrel, and a movable plunger mounted in the barrel and movable to withdraw together with the connector. Thus, when the plunger is pushed into the tube-shaped body of the connector, the connector will be hooked to the distal end of the plunger. Then the connector together with the needle will be pulled into the inside of the barrel when the plunger is withdrawn.

## Description

### BACKGROUND OF THE INVENTION

### 1. Field of the Invention

The present invention relates to a safety syringe, and more particularly to a safety syringe having a retraction mechanism that can be withdrawn back to an inside barrel directly for needle stick protection after syringe operation, without any additional operation to motivate the retraction mechanism.

### 2. Description of the Related Art

A conventional syringe comprises a barrel, a needle firmly seated on an end-portion of the barrel with a tapped adapter, and a plunger inserted into the barrel. When starting to operate the syringe, the plunger is pulled backward to draw the liquid medicine into the barrel. Then, the plunger can be pushed forward to inject the liquid medicine through the needle hole into the human body. However, the needle is protruded outward from the barrel, so that the operator or other medical personnel is easily hurt by the protruding needle during syringe operation.

Another conventional syringe was disclosed in U.S. Patent No. 5,019,044, comprising a barrel, a clamping means having a clamping element to lock a connecting seat, and a spring mounted between the connecting seat and the clamping element of the clamping means.

Another conventional syringe was disclosed in U.S. Patent No. 6,066,115, comprising a barrel, a needle set mounted in the barrel and having a bottom provided with a barb-shaped elastic plate, and a plunger having an end-portion formed with a protruding post having a conical head.

Another conventional syringe was disclosed in U.S. Patent No. 6,712,788-B2, comprising a supporting body, a needle set mounted in the supporting body and having an end formed with a longer tongue and a shorter tongue, and a plunger having an end face formed with an open cylindrical seat.

### SUMMARY OF THE INVENTION

The primary objective of the present invention is to provide a safety syringe having a retraction mechanism that can be pulled directly to retract the needle back to an inside barrel without any additional operation to motivate the retraction mechanism.

Another objective of the present invention is to provide a safety syringe, wherein the needle is withdrawn into the inside barrel after syringe operation, thereby preventing the operator or another medical personnel from being hurt by the protruding needle, so as to provide a protective effect when syringe operation.

A further objective of the present invention is to provide a safety syringe that can be manufactured easily in automatic equipment, thereby facilitating the user using the safety syringe.

A further objective of the present invention is to provide a single use and disposable safety syringe, so that the patient can be reduced the possibility of infection by other diseases.

In accordance with the present invention, there is provided a safety syringe, comprising:
a barrel with an inside seating-ring to receive a connector seating;
a connector securely seated in an end of an inside barrel, which one end-portion formed to a protruding tube with a fluid path in a central tube to adapt for a needle hub, and another end-portion formed to a tube-shaped body having a plurality of resilient leverage plates surrounding the tube wall, each resilient leverage plate being formed a connecting rib on both sides, a protruding seating-flange and releasing-flange on the end-portions separately; and
a moveable plunger mounted in the barrel inside, having a distal end for inserting to the tube-shaped body of the connector when the plunger is pushed forward to the inside barrel, the distal end of the plunger being formed to a column type with the features of the withdraw-ring and the stop-column, the releasing-flanges of the leverage plates being engaged to the withdraw-ring of the plunger and reside on the stop-column of the plunger when the plunger is pushed forward to the end of travel, and the connector together with the needle will be pulled back to the inside barrel when the plunger is withdrawn.

Further benefits and advantages of the present invention will become apparent after a careful reading of the detailed description with appropriate reference to the accompanying drawings.

### BRIEF DESCRIPTION OF THE DRAWINGS

Fig. 1 is a partially perspective view of a safety syringe in accordance with the preferred embodiment of the present invention;
Fig. 2 is a cross-sectional view for a barrel of the safety syringe as shown in Fig. 1;
Fig. 3 is a partially perspective cross-sectional view of a connector of the safety syringe as shown in Fig. 1;
Fig. 4 is a cross-sectional assembly view of the safety syringe as shown in Fig. 1;
Fig. 5 is a schematic operational view of the safety syringe as shown in Fig. 4;
Fig. 6 is a following schematic operational view of the safety syringe as shown in Fig. 5;
Fig. 7 is a following schematic operational view of the safety syringe as shown in Fig. 6;
Fig. 8 is a partially perspective cross-sectional view of a connector of a safety syringe in accordance with another embodiment of the present invention; and
Fig. 9 is a cross-sectional assembly view of the safety syringe in accordance with another embodiment of the present invention.

### DETAILED DESCRIPTION OF THE INVENTION

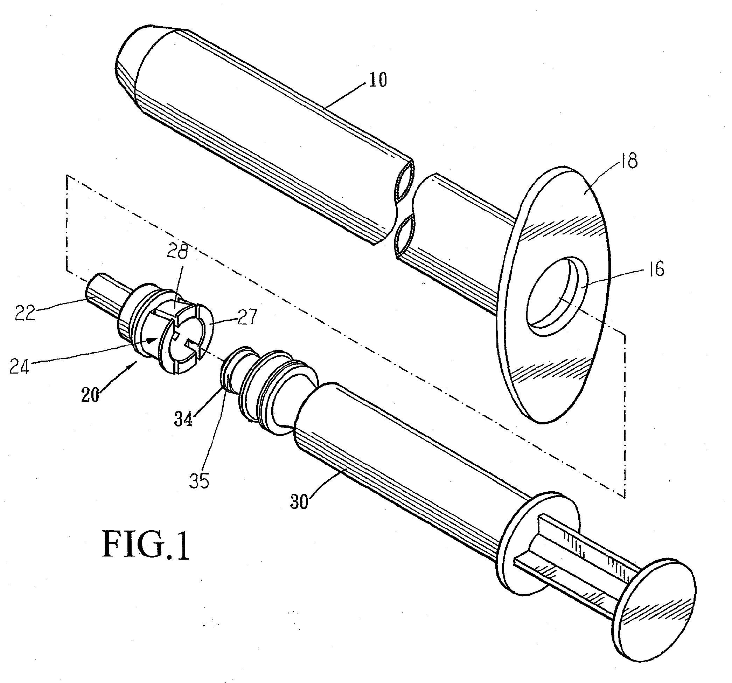

Referring to the drawings and initially to Fig. 1, a safety syringe in accordance with the preferred embodiment of the present invention comprises a barrel 10, a connector 20 seated in the barrel 10, and a plunger 30 inserted to the inside barrel. And on the distal end of the plunger 30 there are two features of withdraw-ring 34 and stop-column 35 are formed to ensure the connector 20 to be pulled back and stored into the barrel inside when the plunger 30 is withdrawn.

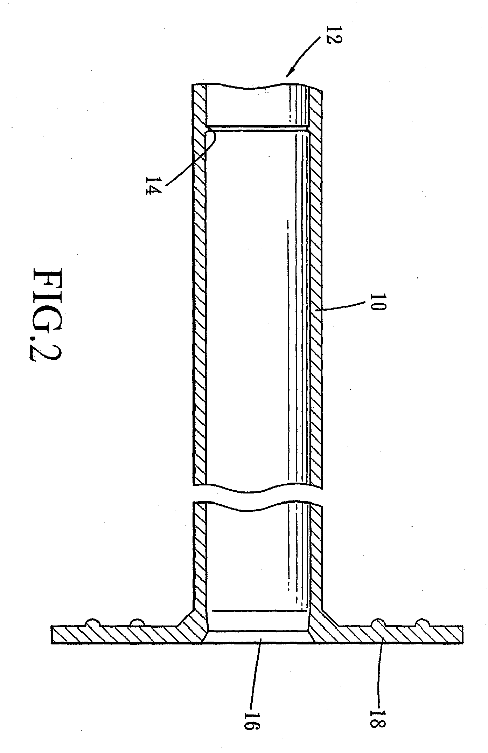

As shown in Fig. 2, the inside of the barrel 10 on an end 12 is formed to a seating-ring 14 which is protruded from one end 12 of the inside barrel for receiving a seating-flanges 282 of the connector 20. On another end 16 of the barrel 10 is formed to an enlarged thumb rest 18 for fingers easily holding when syringe operation.

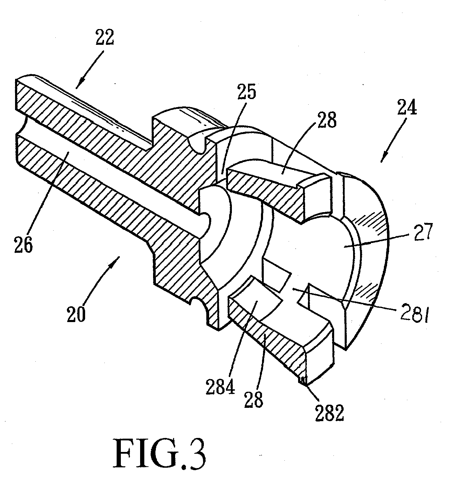

As shown in Fig. 3, a connector 20 on an end-portion is formed to a protruding tube 22 with a fluid path in the central tube 26. Another end-portion is formed to an tube-shaped body 24. Surrounding the peripheral wall of the tube-shaped body 24 is arranged a plurality of resilient leverage plates 28 with the seating-flanges 282 and the releasing-flange 284 protruded on each end, a plurality of fixing plates 27, and a plurality of mediate portion formed to a connecting rib 281 to connect a respective fixing plate 27 and resilient leverage plates 28. And, in the middle of the connector 20 having a gap 25 is formed between the protruding tube 22 and the tube-shaped body 24 for receiving the withdraw-ring 34 of plunger 30.

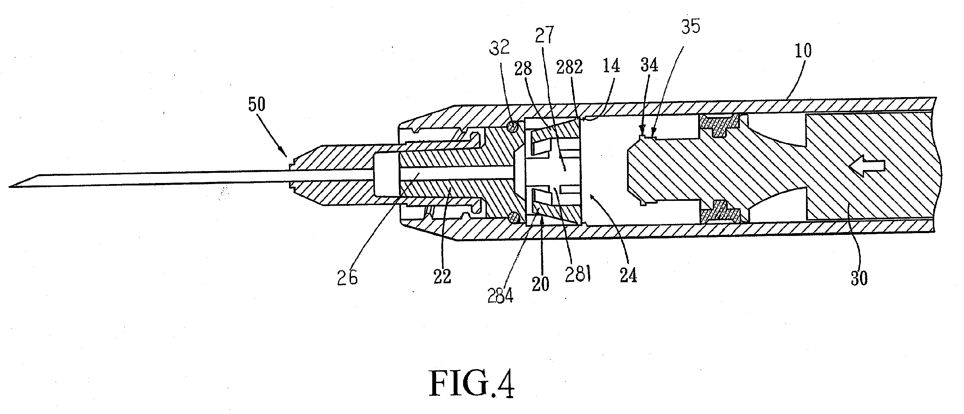

As shown in Fig. 4, the connector 20 with the part of seating-flange 282 to seat on the seating-ring 14 and with the another part of ring-seal 32 fitting in the lumen of barrel 10 to provide a waterproof effect and maintain the connector 20 in a fixed position. On the protruding tube 22 of the connector 20, there is a needle 50 mounted for an injection purpose. When the plunger 30 draws the liquid medicine into the barrel 10 and is pushed forward continuously for injection, the distal end of the plunger 30 is moved into the tube-shaped body 24 of the connector 20.

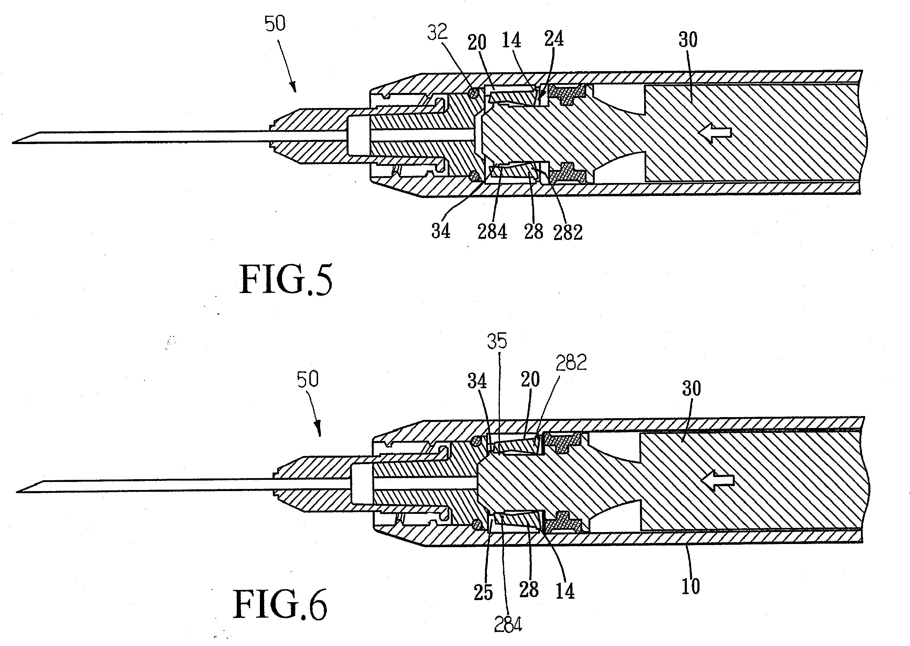

As shown in Fig. 5, the distal end of the plunger 30 is continuously moved in the tube-shaped body 24 of the connector 20. The withdraw-ring 34 of the plunger 30 starts pressing the releasing-flange 284 of the connector 20 upward to make the seating-flange 282 of the connector 20 releasing the seating relation gradually between the seating-flange 282 of the connector 20 and the seating-ring 14 of the barrel 10.

As shown in Fig. 6, when the distal end of plunger 30 reaches to the forward end of travel, the releasing-flange 284 of the connector 20 will climb over the withdraw-ring 34 of the plunger 30 and resides on the stop-column 35 of the plunger 30. Then, the withdraw-ring 34 is dropped into the gap 25 of the connector 20 to engage with the connector 20 together. The stop-column 35 is adjacent to the withdraw-ring 34 and which is formed to an accommodate diameter to ensure the seating-flange 282 of the connector 20 completely releasing from the seating-ring 14 of the barrel 10.

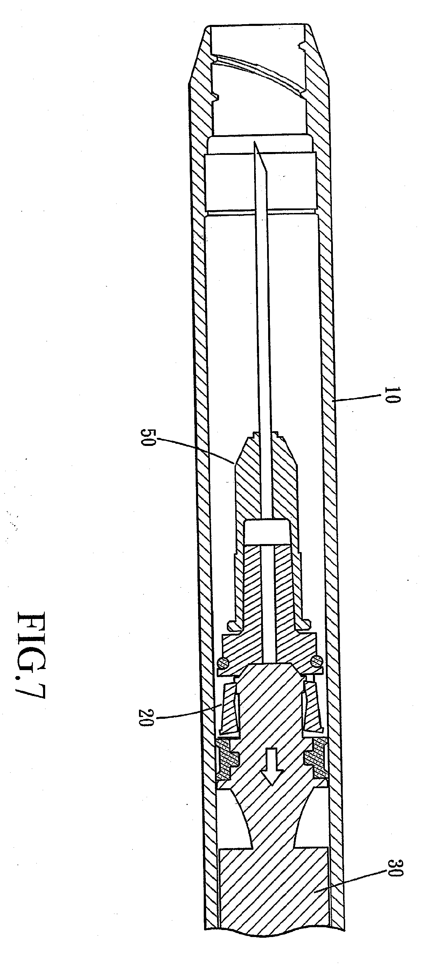

As shown in Fig. 7, when the plunger 30 is withdrawn backward, the withdraw-ring 34 of the plunger 30 will firmly hook to the releasing-flange 284 of the connector 20. Then, the connector 20 together with needle 50 will be pulled back into the lumen of the barrel 10. Thus, the needle 50 is stored and protected by the inside barrel 10, thereby preventing the needle 50 from hurting people accidentally.

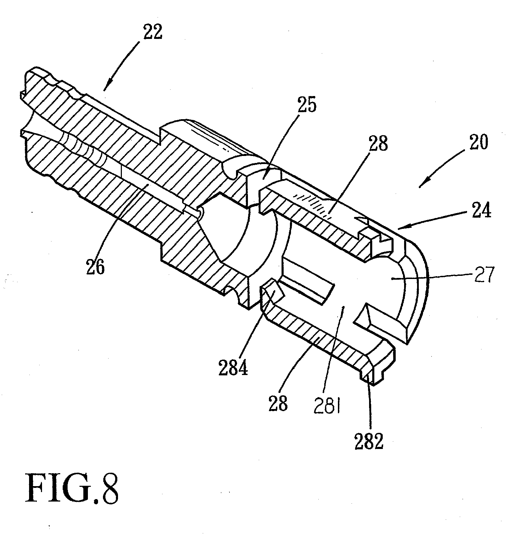

Fig. 8 is a partially perspective cross-sectional view of a connector of a safety syringe in accordance with another embodiment of the present invention. One end-portion of the connector 20 is formed to a protruding tube 22 with a core hole 26 for receiving a size of cannula. Another end-portion is formed to a tube-shaped body 24. Surrounding the peripheral wall of the tube-shaped body 24 is arranged a plurality of resilient leverage plates 28 with seating-flanges 282 and releasing-flange 284 which are protruded on each end, a plurality of fixing plates 27. And also a plurality of mediate portions are formed to a connecting rib 281 to connect a respective fixing plate 27 and resilient leverage plates 28. And, in the middle of the connector 20 having a gap 25 are formed between the protruding tube 22 and the tube-shaped body 24 for receiving the withdraw-ring 34 of the plunger 30.

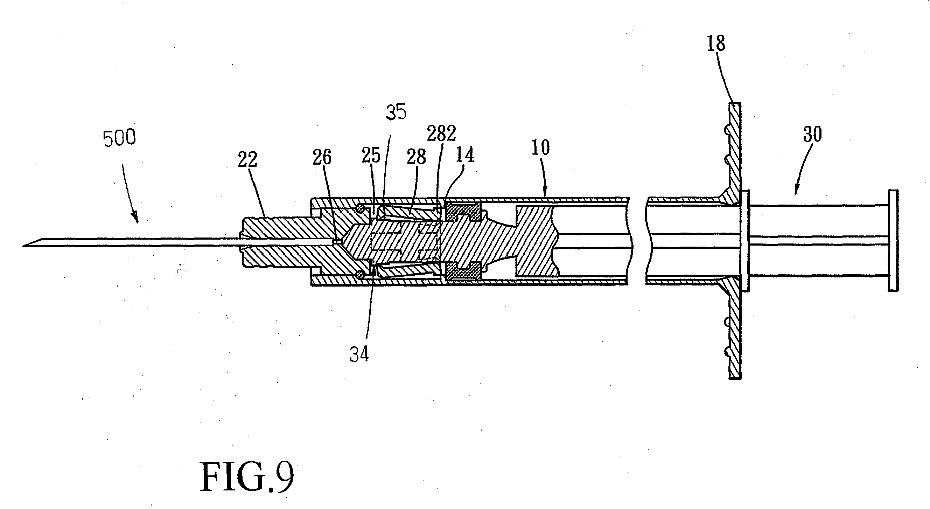

Referring Fig. 9, a size of cannula 500 is mounted on the core hole 26 of the protruding tube 22 in the connector 20. The connector 20 with the part of the seating-flange 282 to seat on the seating-ring 14 and with another part of the ring-seal 32 fitting in the lumen of barrel 10 to provide a waterproof effect and maintain the connector 20 in a fixed position. On the protruding tube 22 of the connector 20, there is a needle 50 mounted for an injection purpose. When the plunger 30 draws the liquid medicine into the barrel 10 and is pushed forward continuously for injection, the distal end of the plunger 30 is moved into the tube-shaped body 24 of the connector 20.

When syringe injection operation, the distal end of the plunger 30 is continuously moved in the tube-shaped body 24 of the connector 20. The withdraw-ring 34 of the plunger 30 starts pressing the releasing-flange 284 of the connector 20 upward to make the seating-flange 282 of the connector 20 releasing the seating relation gradually between the seating-flange 282 of the connector 20 and the seating-ring 14 of the barrel 10. When the distal end of plunger 30 reaches to the forward end of travel, the releasing-flange 284 of the connector 20 will climb over the withdraw-ring 34 of the plunger 30 and resides on the stop-column 35 of the plunger 30. Then the withdraw-ring 34 is dropped into the gap 25 of the connector 20 to engage with the connector 20 together. The stop-column 35 is adjacent to the withdraw-ring 34 and which is formed to an accommodate diameter to ensure the seating-flange 282 of the connector 20 completely releasing from the seating-ring 14 of the barrel 10. When the syringe injection is finished, the plunger 30 is withdrawn backward, the withdraw-ring 34 of the plunger 30 will firmly hook to the releasing-flange 284 of the connector 20. Then the connector 20 together with needle 50 will be pulled back into the lumen of the barrel 10. Thus, the needle 50 is stored and protected by the inside barrel 10, thereby preventing the needle 50 from hurting people accidentally.

Although the invention has been explained in relation to its preferred embodiment(s) as mentioned above, it is to be understood that many other possible modifications and variations can be made without departing from the scope of the present invention. It is, therefore, contemplated that the appended claim or claims will cover such modifications and variations that fall within the true scope of the invention.

## Claims

1. A safety syringe, comprising:
a barrel, on an end is formed with a seating-ring that protruded from an inside barrel for seating a seating-flange of a connector;
a connector, on an end-portion is formed with a protruding tube with a fluid path in a central tube, another end-portion is formed to a tube-shaped body, surrounding a peripheral wall of the tube-shaped body is arranged a plurality of resilient leverage plates with seating-flanges and releasing-flange protruded on each end, a plurality of fixing plates, and a plurality of mediate portions formed to a connecting rib to connect a respective fixing plate and resilient leverage plates, and, in the middle of the connector having a gap is formed between the protruding tube and the tube-shaped body for receiving the withdraw-ring of the plunger; and
a plunger, mounted in the barrel and having a distal end to form with a withdraw-ring and a stop-column, the distal end of the plunger moves in the
tube-shaped body of the connector, then the withdraw-ring presses the releasing-flange of the connector upward to make the seating-flange of the connector releasing the seating relation between the seating-flange of the connector and the seating-ring of the barrel, and, when the plunger is withdrawn backward, the withdraw-ring firmly hooks the releasing-flange of the connector, then the connector together with the needle will be pulled back into the lumen of barrel.

2. The safety syringe in accordance with claim 1, wherein the inside of the barrel has an end-portion formed with a seating-ring for receiving the seating-flanges of the connector, the connector with an end-portion to form to a tube-shaped body with a seating-flange extended outward for resting on the seating-ring of barrel.

3. The safety syringe in accordance with claim 1, wherein an end-portion of leverage plates having a formed with releasing-flange, and the distal end of the plunger is moved to press the releasing-flange of the leverage plates of the connector, so that the leverage plates are pivoted to perform a leverage motion.

4. The safety syringe in accordance with claim 3, wherein the distal end of the plunger is pushed on the releasing-flange of the leverage plates in the connector to pivot the leverage plates to release the seating relation between the seating-flanges of the connector and the seating-ring of the barrel.

5. The safety syringe in accordance with claim 3, wherein the distal end of the plunger to form with a withdraw-ling to press the releasing-flange of the leverage plates of the connector.

6. The safety syringe in accordance with claim 3, wherein the distal end of the plunger to form with a stop-column to reside the releasing-flange of the leverage plates of the connector, so that the connector enables to completely release the seating relation from the barrel when the plunger is withdrawn backward.

7. The safety syringe in accordance with claim 6, wherein the withdraw-ring of the plunger is hooked on the releasing-flange of the leverage plates of the connector when the plunger is withdrawn backward.

8. The safety syringe in accordance with claim 1, wherein the leverage plates are arranged on a tube-shaped peripheral wall of the connector and the connecting-rib are formed on the both sides of the leverage plates.

9. The safety syringe in accordance with claim 1, wherein the connector has a tube-shaped peripheral wall formed with a plurality of fixing plates and leverage plates, a mediate portion formed with a connecting rib to connect to a respective one of the fixing plates, so that the leverage plates are pivoted to the fixing plates with a connecting rib to perform a leverage motion.

10. The safety syringe in accordance with claim 1, further comprising a needle mounted on the protruding tube of the connector and protruded outward from the protruding tube of the connector.

11. The safety syringe in accordance with claim 1, further comprising a cannula mounted on the protruding tube of the connector and protruded outward from the protruding tube of the connector.

12. The safety syringe in accordance with claim 1, wherein the connector is released from the barrel and is combined with the plunger by a leverage motion of the leverage plates of the connector.
